# FASCICULE DE BREVET EUROPEEN

(11) **EP 1 001 923 B1**
(45) Date de publication et mention de la délivrance du brevet: **18.12.2002**
(21) Numéro de dépôt: 98945160.4
(22) Date de dépôt: 31.07.1998
(51) Int. Cl.: C07C 17/278, C07C 19/01

(54) **PROCEDE DE PREPARATION D'HYDROCARBURES HALOGENES**
VERFAHREN ZUR HERSTELLUNG VON HALOGENIERTEN KOHLENWASSERSTOFFEN
METHOD FOR PREPARING HALOGENATED HYDROCARBONS

(30) Priorité: 08.08.1997 BE 9700669; 16.07.1998 BE 9800546
(43) Date de publication de la demande: 24.05.2000
(73) Titulaire: SOLVAY (Société Anonyme), 1050 Bruxelles (BE)
(72) Inventeur: MATHIEU, Véronique, B-1300 Wavre (BE); JANSSENS, Francine, B-1800 Vilvoorde (BE)
(74) Mandataire: Jacques, Philippe
(86) Numéro de dépôt international: EP9804966
(87) Numéro de publication internationale: WO99007659

(56) Documents cités:
- EP-A- 0 787 707
- WO-A-97/07083
- US-A- 3 862 978

## Description

La présente invention concerne un procédé de préparation d'hydrocarbures halogénés comprenant au moins 3 atomes de carbone par réaction catalytique entre un haloalcane et une oléfine halogénée.

L'addition d'un haloalcane sur une oléfine halogénée est une réaction bien connue. Cependant, il est parfois difficile de contrôler la réaction de telle sorte qu'une seule molécule d'oléfine halogénée s'additionne à une molécule d'haloalcane (formation d'un produit d'addition ou adduit 1:1).

Il est généralement admis qu'un rapport molaire haloalcane/oléfine halogénée au moins égal à deux est requis pour obtenir une sélectivité élevée en produits d'addition 1:1 (voir par exemple T. Asahara et al., Kogyo Kagaku Zasshi, 72 (1969), 1526-9; et M. Belbachir et al., Makromol.Chem., 185 (1984), 1583-95 et la demande de brevet PCT 95/04021 ). Toutefois, cet excès d'haloalcane réduit la productivité volumique du procédé de synthèse et rend malaisée la séparation des produits de réaction.

D'autre part, il est de pratique courante d'utiliser un solvant pour dissoudre convenablement le catalyseur et augmenter le rendement de la réaction, bien que ce faisant, on limite également la productivité volumique du procédé. Classiquement, les nitriles sont utilisés à cette fin, en particulier l'acétonitrile. Ainsi, la demande de brevet PCT 96/01797, qui concerne un procédé de préparation d'hydrofluorocarbures (HFC) au départ de précurseurs chlorés, décrit la préparation de 1,1,1,3,3-pentachloropropane par télomérisation entre du tétrachlorométhane (CCl₄) et du chlorure de vinyle (VC), en présence d'acétonitrile. Il en résulte une faible productivité volumique (de l'ordre de 0.1 mole/h.kg), même avec un rapport CCl₄ / VC de 1,7. La présence d'un nitrile est également envisagée dans le procédé de fabrication d'un hydrocarbure halogéné par réaction entre un haloalcane et un haloalcène décrit dans la demande de brevet PCT 97/07083. En outre, tous les exemples rapportent des rapports molaires haloalcane / haloalcène de 4 ou plus. Les productivités qui en découlent sont par conséquent faibles.

L'invention vise dès lors à fournir un procédé de préparation d'hydrocarbures halogénés comprenant au moins 3 atomes de carbone, qui ne présente plus les inconvénients des procédés connus, en particulier un procédé dans lequel on obtient le produit d'addition 1:1 avec une sélectivité et une productivité volumique élevées, sans dilution du milieu réactionnel, ce qui facilite en outre les étapes ultérieures de séparation des produits réactionnels.

En conséquence, la présente invention concerne un procédé de préparation d'hydrocarbures halogénés comprenant au moins 3 atomes de carbone par réaction catalytique, en l'absence substantielle de nitrile, entre un haloalcane et une oléfine halogénée dans un rapport molaire haloalcane / oléfine halogénée inférieur à 2.

Le procédé selon l'invention peut être réalisé en continu ou en discontinu. Il est entendu que les rapports molaires entre les réactifs sont exprimés, dans un procédé en discontinu, entre les quantités totales de réactifs mises en oeuvre et, dans un procédé en continu, entre les quantités stationnaires de réactifs présentes dans le réacteur.

Selon la présente invention, le rapport molaire entre l'haloalcane et l'oléfine halogénée est inférieur à 2. Ce rapport est en général égal ou supérieur à 0,7. Avantageusement, ce rapport est égal ou supérieur à 0,8. D'une manière préférée, il est supérieur ou égal à 1. D'excellents résultats sont obtenus lorsque ce rapport est d'au moins 1,2. On a en effet observé de manière surprenante dans le procédé selon l'invention, qu'il est possible de travailler avec un rapport entre l'haloalcane et l'oléfine proche de la stoechiométrie sans affecter de manière sensible la sélectivité.

Le procédé selon l'invention est effectué en l'absence substantielle de composés organiques ayant un groupement nitrile (C≡N). Par absence substantielle, on entend une teneur en poids, par rapport à l'ensemble du mélange réactionnel, de moins de 2 %, de préférence de moins de 1 %, de manière particulièrement préférée de moins de 0,1 %. Plus particulièrement, le procédé selon l'invention est effectué en l'absence substantielle d'acétonitrile.

Les haloalcanes engagés dans le procédé selon la présente invention sont en général des composés organiques saturés. Ils possèdent de préférence de un à trois atomes de carbone et de préférence au moins 2 atomes de chlore. Ils peuvent également comprendre d'autres substituants tels que d'autres atomes d'halogène, des groupes alkyle ou halogénoalkyle. A titre d'exemples d'haloalcanes selon la présente invention, on peut citer le dichlorométhane, le chloroforme, le tétrachlorure de carbone, le 1,1,1-trichloroéthane, le 1,1,1-trichloro-2,2,2-trifluoroéthane. Le tétrachlorure de carbone est tout particulièrement préféré.

Les oléfines halogénées engagées dans le procédé selon la présente invention sont en général des dérivés d'un haloéthène ou d'un haloproprène, et répondent de préférence à la formule 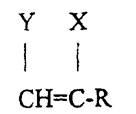 avec Y=H ou Cl, X=Cl ou F et R=H, Cl, F, CH₃. Parmi ces composés, ceux où Y=H, X=Cl et R=H ou CH₃ sont préférés.

Les hydrocarbures halogénés obtenus selon le procédé de la présente invention appartiennent, en général, à la famille des chloropropanes, des chlorobutanes ou des chloropentanes. Les atomes de carbone desdits chloropropanes, chlorobutanes et chloropentanes peuvent également être substitués par d'autres groupes fonctionnels tels que d'autres atomes d'halogène (comme des atomes de brome ou d'iode), des groupes alkyle ou halogénoalkyle, des groupes nitrile (C≡N) ou acide carboxylique (COOH). Les chloropropanes et les chlorobutanes non substitués par d'autres groupes fonctionnels sont préférés.

De préférence, les hydrocarbures halogénés obtenus selon le procédé de la présente invention répondent à la formule générale CₙH₍₂ₙ₊₂₎₋ₚClₚ dans laquelle n est un nombre entier et possède les valeurs 3 ou 4, p est un nombre entier qui possède les valeurs 3 à 7. Des exemples de composés obtenus selon le procédé de la présente invention sont le 1,1,1,3,3-pentachloropropane, le 1,1,1,3,3-pentachlorobutane, le 1,1,1,3-tétrachloropropane, le 1,1,3,3-tétrachlorobutane, le 1,1,1,3,3,3-hexachlororopropane et le 1,1-dichloro-2-trichlorométhylpropane. Parmi ces composés, le 1,1,1,3,3-pentachloropropane, le 1,1,1,3,3-pentachlorobutane, le 1,1,1,3,3,3-hexachloropropane et le 1,1-dichloro-2-trichlorométhylpropane sont préférés. Le 1,1,1,3,3-pentachlorobutane et le 1,1,1,3,3-pentachloropropane sont tout particulièrement préférés.

Les hydrocarbures halogénés obtenus selon le procédé de l'invention sont des précurseurs des analogues fluorés correspondants, lesquels peuvent être facilement obtenus par traitement avec du fluorure d'hydrogène en présence d'un catalyseur tel qu'un sel d'antimoine, un sel de titane, un sel de tantale ou un sel d'étain.

Le système catalytique utilisé dans la présente invention comprend de préférence au moins un composé du cuivre. Avantageusement, il s'agit d'un composé de cuivre (II). De manière particulièrement préférée, ce composé de Cu (II) est choisi parmi le chlorure de cuivre (II), l'hydroxychlorure de cuivre (II), l'acétylacétonate de cuivre (II) et l'hexafluoroacétylacétonate de Cu (II) et leurs mélanges. D'excellents résultats ont été obtenus avec l'hexafluoroacétyl-acétonate de Cu (II).

Le rapport molaire entre le composé de Cu (II) et l'oléfine est habituellement supérieur ou égal à 0,0001. Avantageusement, il est supérieur ou égal à 0,001. De préférence, il est supérieur ou égal à 0,005. Le rapport molaire entre le composé de Cu (II) et l'oléfine est habituellement inférieur ou égal à 1. Avantageusement, il est inférieur ou égal à 0,5. De préférence, il est inférieur ou égal à 0,1.

Le système catalytique utilisé dans la présente invention comprend de préférence un cocatalyseur, en particulier une amine, un amide ou un oxyde de trialkylphosphine. Comme amides utilisables comme cocatalyseur, on peut citer la N-méthylpyrrolidone et le N,N-diméthylformamide. Comme oxydes de trialkylphosphine utilisables comme cocatalyseur, on peut citer les composés de formule (R₁R₂R₃)PO, dans laquelle R₁, R₂ et R₃ représentent des groupements alkyles en C3-C10, identiques ou différents, de préférence linéaires. On retient en particulier l'oxyde de tri-(n-butyl)phosphine, l'oxyde de tri-(n-hexyl)-phosphine, l'oxyde de tri-(n-octyl)phosphine, l'oxyde de n-octyl-di-(n-hexyl)-phosphine, l'oxyde de n-hexyl-di-(n-octyl)-phosphine oxyde et leurs mélanges. On préfère utiliser une amine comme cocatalyseur, en particulier une amine primaire. Sont préférées les amines aliphatiques comprenant de 3 à 25 atomes de carbone. Sont particulièrement préférées les amines aliphatiques comprenant de 3 à 22 atomes de carbone. Comme amines aliphatiques primaires utilisables dans le procédé selon l'invention, on peut citer la n.propylamine, l'isopropylamine, la n.butylamine, l'isobutylamine, la t.butylamine, la pentylamine et l'isoamylamine. Parmi ces amines, une préférence toute particulière est accordée aux amines dont la chaîne alkyle est ramifiée et plus spécialement aux amines tert-alkyle répondant la formule générale (I) 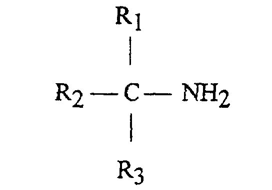 dans laquelle R₁, R₂ et R₃ représentent des groupements alkyle en C1-C8. Des amines répondant la formule (I) sont notamment la t.butylamine, et les amines tert-alkyles PRIMENE® 81-R et JM-T commercialisées par Rohm and Haas Company. La t.butylamine est tout particulièrement préférée.

Le système catalyseur-cocatalyseur préféré selon la présente invention est le système composé d'un composé de cuivre (II) formé avec un composé organique acide et une amine primaire dont l'atome de carbone voisin du groupe NH₂ est un atome de carbone quaternaire, c'est-à-dire dépourvu d'atome d'hydrogène. Est particulièrement préféré, le système catalyseur-cocatalyseur formé par l'acétylacétonate de cuivre (II) et la t.butylamine.

Le rapport molaire entre le cocatalyseur et l'oléfine est généralement supérieur ou égal à 0,01. De préférence, ce rapport molaire est supérieur ou égal à 0,05. Avantageusement, ce rapport molaire est supérieur ou égal à 0,1. Toutefois, ce rapport molaire est habituellement inférieur ou égal à 2. De préférence, ce rapport molaire est inférieur ou égal à 1. Avantageusement, ce rapport molaire est inférieur ou égal à 0,5. La quantité de cocatalyseur mise en oeuvre peut varier, sur une base molaire, d'environ 0,1 à environ 25 fois la quantité de catalyseur, de préférence d'environ 0,5 à environ 20 fois.

Il est entendu que les rapports ci-dessus catalyseur / oléfine et cocatalyseur / oléfine sont exprimés, dans un procédé en discontinu, par rapport à la quantité totale d'oléfine mise en oeuvre et, dans un procédé en continu, par rapport à la quantité stationnaire d'oléfine présente dans le réacteur.

Généralement, la réaction se déroule à une température supérieure ou égale à la température ambiante. De préférence, la température est supérieure ou égale à 50 °C. Avantageusement, la température est supérieure ou égale à 70 °C. Toutefois, la température est généralement inférieure ou égale à 200 °C. De préférence, la température est inférieure ou égale à 175 °C. Avantageusement, la température est inférieure ou égale à 150 °C. Une préférence toute particulière est montrée pour une température inférieure ou égale à 120 °C, voire 100 °C.

La durée de la réaction dans un procédé en discontinu ou le temps de séjour dans un procédé en continu sont fonction de divers paramètres tels que la température de réaction, la concentration en réactifs et en catalyseur dans le mélange réactionnel et leurs rapports molaires. En général, en fonction de ces paramètres, le temps de séjour ou la durée de réaction peuvent varier de 5 minutes à 10 heures. Avantageusement, dans un procédé en discontinu, le temps de réaction est généralement supérieur ou égal à 30 minutes avec une préférence pour les temps de réaction supérieurs ou égaux à 60 minutes. Toutefois, le temps de réaction est habituellement inférieur ou égal à 10 heures avec une préférence pour les temps de réaction inférieurs ou égaux à 8 heures.

La pression est généralement choisie de façon à maintenir le milieu réactionnel en phase liquide. La pression mise en oeuvre varie en fonction de la température du milieu réactionnel. La pression est habituellement supérieure ou égale à la pression atmosphérique et inférieure ou égale à 10 bars.

Les exemples ci-après illustrent l'invention de manière non limitative.

### Exemples 1-6

On a préparé du 1,1,1,3,3-pentachloropropane au départ de chlorure de vinyle (VC) et de tétrachlorure de carbone, par réaction entre ces réactifs en présence d'un composé du cuivre (II) et d'une amine ou d'acétonitrile. Pour ce faire, on a introduit les réactifs, le catalyseur et l'amine ou l'acétonitrile dans un autoclave de 300 ml dont les parois internes sont recouvertes de téflon. L'appareil a ensuite été fermé hermétiquement, placé dans un four vertical et la température a été augmentée progressivement et maintenue à 90 °C pendant la durée de la réaction. L'agitation a été assurée par un barreau magnétique placé dans le fond de l'autoclave. En fin de réaction, on a laissé refroidir l'autoclave, un échantillon de liquide a été prélevé à la seringue et dosé par une méthode chromatographique pour déterminer le taux de conversion de l'oléfine et la sélectivité en hydrocarbure halogéné (produit 1+1). Les résultats obtenus sont rassemblés au tableau 1.

### Exemple 7-10

On a répété la procédure expérimentale des exemples précédents avec du 2-chloroprop-1-ène (2-CPe) au lieu de VC, en présence d'acétylacétonate de cuivre (II) comme catalyseur et de tert-butylamine comme cocatalyseur, pendant une durée de réaction de 0.5 h à 90 °C. Les résultats obtenus figurent également dans le tableau II.

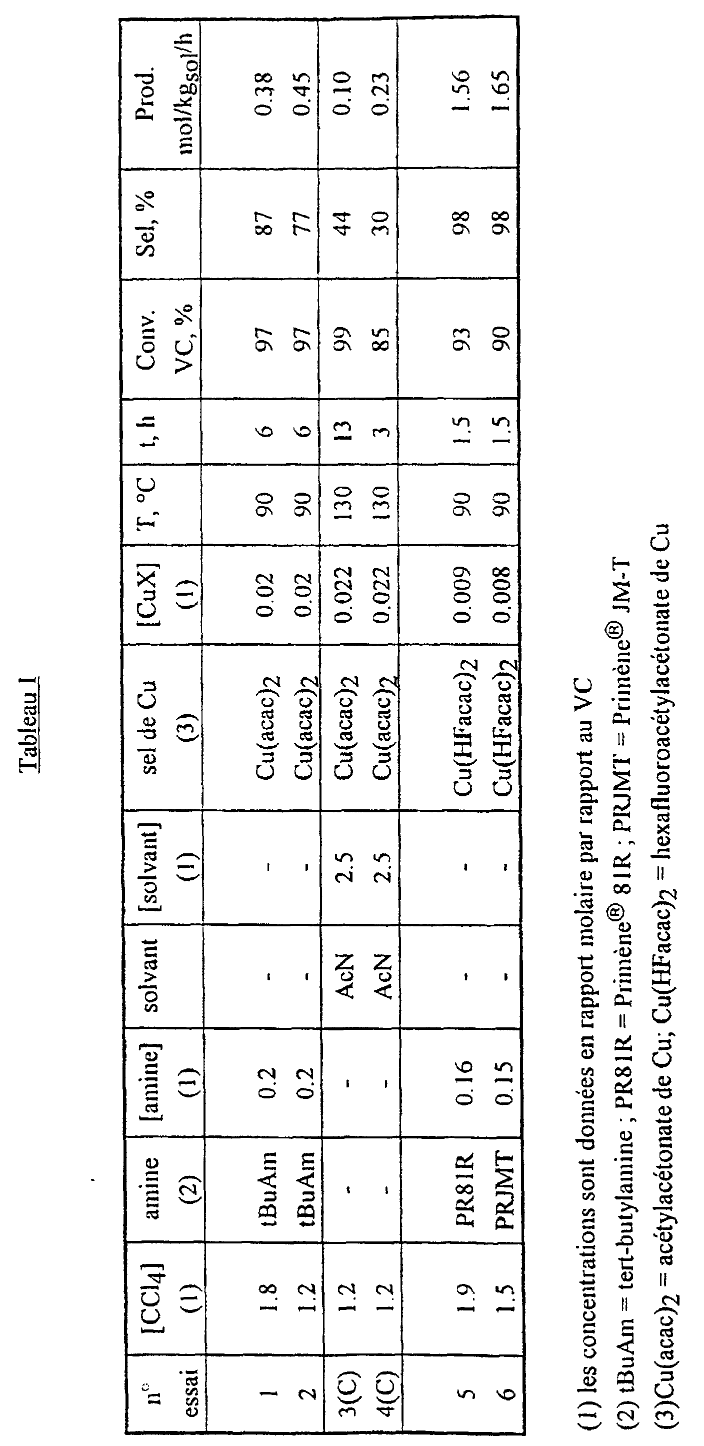

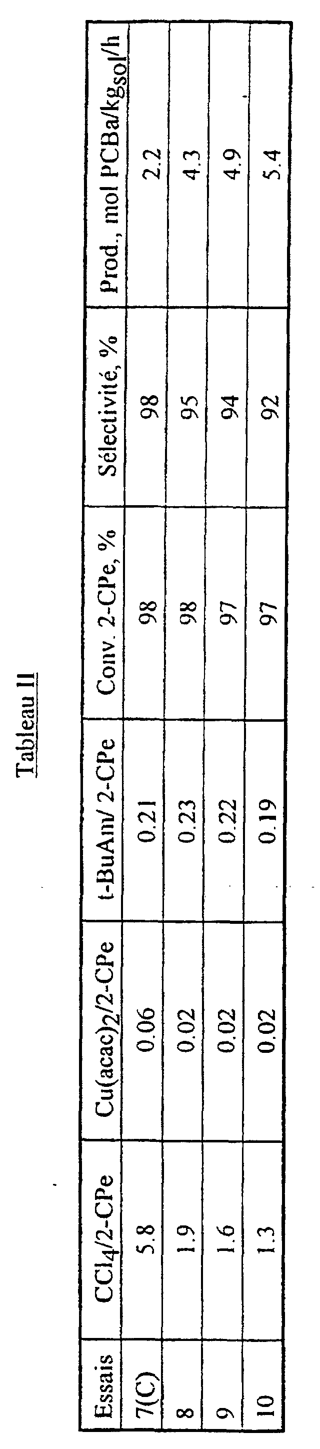

## Revendications

1. Procédé de préparation d'hydrocarbures halogénés comprenant au moins 3 atomes de carbone par réaction catalytique entre un haloalcane et une oléfine halogénée, **caractérisé en ce qu'**on effectue la réaction en l'absence substantielle de nitrile et avec un rapport molaire entre l'haloalcane et l'oléfine halogénée inférieur à 2.

2. Procédé selon la revendication 1 dans lequel le rapport molaire entre l'haloalcane et l'oléfine halogénée est supérieur ou égal à 0,7.

3. Procédé selon l'une quelconque des revendications précédentes dans lequel l'oléfine halogénée répond à la formule 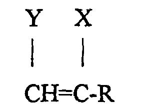 où Y représente H ou Cl, X représente Cl ou F et R représente H, Cl, F, CH₃.

4. Procédé selon la revendication 3 où Y représente H, X représente Cl et R représente H ou CH₃.

5. Procédé selon l'une quelconque des revendications précédentes dans lequel le système catalytique comprend au moins un composé du cuivre.

6. Procédé selon la revendication 5 dans lequel le composé du cuivre est un composé de cuivre (II).

7. Procédé selon la revendication 6 dans lequel le composé du cuivre est choisi parmi le chlorure de cuivre (II),l'hydroxychlorure de cuivre (II), l'acétylacétonate de cuivre (II), l'hexafluoroacétylacétonate de Cu (II) et leurs mélanges.

8. Procédé selon l'une quelconque des revendications précédentes dans lequel le système catalytique comprend un cocatalyseur.

9. Procédé selon la revendication 8 dans lequel le cocatalyseur est une amine.

10. Procédé selon la revendication 9 dans lequel l'amine est une amine aliphatique primaire comprenant de 3 à 25 atomes de carbone dont la chaîne alkyle est ramifiée.

11. Procédé selon l'une quelconque des revendications précédentes réalisé en continu.

12. Procédé de préparation d'un hydrocarbure fluoré comprenant au moins 3 atomes de carbone, selon lequel
(a) on prépare un hydrocarbure halogéné comprenant au moins 3 atomes de carbone selon le procédé de l'une quelconque des revendications 1 à 11 ;
(b) on soumet l'hydrocarbure halogéné obtenu à l'étape (a) à un traitement avec du fluorure d'hydrogène en présence d'un catalyseur.

## Patentansprüche

1. Verfahren zur Herstellung von halogenierten Kohlenwasserstoffen, die wenigstens 3 Kohlenstoffatome aufweisen, durch katalytische Umsetzung zwischen einem Halogenalkan und einem halogenierten Olefin, **dadurch gekennzeichnet, daß** man die Umsetzung im wesentlichen in Abwesenheit von Nitril und mit einem Molverhältnis von Halogenalkan zu halogeniertem Olefin von kleiner als 2 ausführt.

2. Verfahren nach Anspruch 1, worin das Molverhältnis von Halogenalkan zu halogeniertem Olefin größer als oder gleich 0,7 ist.

3. Verfahren nach einem der vorstehenden Ansprüche, worin das halogenierte Olefin der Formel 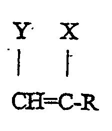 entspricht, worin Y für H oder Cl steht, X für Cl oder F steht und R die Bedeutung H, Cl, F, CH₃ hat.

4. Verfahren nach Anspruch 3, worin Y für H steht, X für Cl steht und R die Bedeutung H oder CH₃ hat.

5. Verfahren nach einem der vorstehenden Ansprüche, worin das katalytische System wenigstens eine Kupferverbindung umfaßt.

6. Verfahren nach Anspruch 5, worin die Kupferverbindung eine Kupfer(II)verbindung ist.

7. Verfahren nach Anspruch 6, worin die Kupferverbindung unter Kupfer(II)chlorid, Kupfer(II)hydroxychlorid, Kupfer(II)acetylacetonat, Kupfer(II)hexafluoracetylacetonat und deren Gemischen ausgewählt wird.

8. Verfahren nach einem der vorstehenden Ansprüche, worin das katalytische System einen Cokatalysator umfaßt.

9. Verfahren nach Anspruch 8, worin der Cokatalysator ein Amin ist.

10. Verfahren nach Anspruch 9, worin das Amin ein aliphatisches primäres Amin mit einem Gehalt an 3 bis 25 Kohlenstoffatomen ist, dessen Alkylkette verzweigt ist.

11. Verfahren nach einem der vorstehenden Ansprüche, das kontinuierlich ausgeführt wird.

12. Verfahren zur Herstellung eines fluorierten Kohlenwasserstoffes, der wenigstens 3 Kohlenstoffatome aufweist, wonach man
(a) einen halogenierten Kohlenwasserstoff mit einem Gehalt an wenigstens 3 Kohlenstoffatomen nach dem Verfahren eines der Ansprüche 1 bis 11 herstellt;
(b) den in Stufe (a) erhaltenen halogenierten Kohlenwasserstoff einer Behandlung mit Fluorwasserstoff in Gegenwart eines Katalysators unterzieht.

## Claims

1. Process for the preparation of halohydrocarbons comprising at least 3 carbon atoms, by catalytic reaction between a haloalkane and a haloolefin, **characterized in that** the reaction is carried out in the substantial absence of nitrile and with a molar ratio between the haloalkane and the haloolefin of less than 2.

2. Process according to Claim 1, in which the molar ratio between the haloalkane and the haloolefin is greater than or equal to 0.7.

3. Process according to either of the preceding claims, in which the haloolefin corresponds to the formula 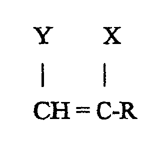 in which Y represents H or Cl, X represents Cl or F and R represents H, Cl, F or CH₃.

4. Process according to Claim 3, in which Y represents H, X represents Cl and R represents H or CH₃.

5. Process according to any one of the preceding claims, in which the catalytic system comprises at least one copper compound.

6. Process according to Claim 5, in which the copper compound is a copper (II) compound.

7. Process according to Claim 6, in which the copper compound is chosen from copper (II) chloride, copper (II) hydroxychloride, copper (II) acetylacetonate and copper (II) hexafluoroacetylacetonate, and mixtures thereof.

8. Process according to any one of the preceding claims, in which the catalytic system comprises a cocatalyst.

9. Process according to Claim 8, in which the cocatalyst is an amine.

10. Process according to Claim 9, in which the amine is a primary aliphatic amine comprising from 3 to 25 carbon atoms in which the alkyl chain is branched.

11. Process according to any one of the preceding claims, which is carried out continuously.

12. Process for the manufacture of a fluorinated hydrocarbon comprising at least 3 carbon atoms according to which
(a) a halogenated hydrocarbon comprising at least 3 carbon atoms is prepared according to the process according to anyone of claims 1 to 11
(b) the halogenated hydrocarbon obtained in step (a) is subjected to a treatment with hydrogen fluoride in the presence of a catalyst.
